# EUROPÄISCHE PATENTANMELDUNG

(11) **EP 2 469 434 A1**
(43) Veröffentlichungstag der Anmeldung: **27.06.2012**
(21) Anmeldenummer: 10197068.9
(22) Anmeldetag: 27.12.2010
(51) Int. Cl.: G06F 19/00

(54) **Verfahren und Einrichtung zur Anzeige von medizinischen Bilddaten**

(71) Anmelder: Siemens Aktiengesellschaft, 80333 München (DE)
(72) Erfinder: Dorn, Karlheinz, 90562, Kalchreuth (DE); Ukis, Vladyslav, 90491, Nürnberg (DE)

(57) **Zusammenfassung**

Zur Anzeige von medizinischen Bilddaten ist vorgesehen, in einem zentralen Datencenter (2) aus mindestens einem medizinischen Eingangsbilddatensatz (E), der in einem dem DICOM-Standard entsprechenden Format oder einem vergleichbaren Datenformat für medizinische Bilddaten vorliegt, eine Hierarchiegruppe von pyramidentransformierten Bildkacheln (K) zu erzeugen, wobei die Bildkacheln (K) in einem Bildkachelspeicher (26) des Datencenters (2) hinterlegt werden. Jeder Bildkachel (K) wird eine URL zugewiesen, die eine Information über die Bildauflösung der Bildkachel (K) und den von Ihr abgedeckten Bildausschnitt enthält. Weiterhin ist vorgesehen, an einem mit dem Datencenter (2) datenkommunikationstechnisch verbundenen Client (3a-3e) mittels eines Web-Browsers eine Internet-Applikation (17) auszuführen, wobei durch die Internet-Applikation (17) ein anzuzeigender Bildbereich sowie eine zugehörige Bildauflösung bestimmt werden. Durch die Internet-Applikation (17) wird dabei die URL der oder jeder Bildkachel (K) mit entsprechender Bildauflösung bestimmt, deren Bildausschnitt mit dem anzuzeigenden Bildbereich überlappt. Unter Zugriff auf die oder jede bestimmte URL werden dann durch die Internet-Applikation (17) die zugehörigen Bildkacheln (K) aus dem Bildkachelspeicher (26) in den Client (3a-3e) geladen und dort angezeigt.

## Beschreibung

Die Erfindung bezieht sich auf ein Verfahren zur Anzeige von medizinischen Bilddaten. Die Erfindung bezieht sich des Weiteren auf eine Einrichtung zur Durchführung des Verfahrens.

Computergestützte Systeme zur Anzeige von digitalen medizinischen Bilddaten sowie zur Unterstützung der Auswertung (Befundung) solcher Bilddaten werden heute in vielen Bereichen der Medizin eingesetzt, insbesondere in der Pathologie sowie in der Mammographie.

Heutige Pathologie-Systeme ermöglichen insbesondere das Einlesen von sogenannten Whole-Slice-Images (WSI), die durch Digitalisieren von mikroskopisch aufgenommenen Dünnschnitten entstehen. Heutige Mammographiesysteme bieten des Weiteren häufig eine sogenannte "Double Reading"-Funktion an, bei der medizinische Studien, d.h. Bilder oder Bilderserien, nach Durchsicht und Auswertung durch einen ersten Nutzer automatisch an einen zweiten Nutzer zur Überprüfung und Verifizierung der Befunde übermittelt werden. Bei dem ersten Nutzer handelt es sich hierbei oft um einen virtuellen Nutzer, d.h. um einen Algorithmus, der die betreffenden Studien automatisch auswertet.

Zudem stellen die jeweils eingesetzten Systeme in der Regel computergestützte Methoden (Tools) zur Unterstützung der Diagnosestellung, des sogenannten "Reading", sowie zum Laden und Lesen von medizinischen Studien und Berichten zur Verfügung. Eine spezielle Funktion mancher Systeme ist insbesondere das sogenannte "Multi-Modality-Reading". Dieser Begriff bezeichnet die Fähigkeit einer einzigen Softwareapplikation, Bilddaten verschiedener bildgebender Modalitäten, wie z.B. Magnetresonanztomographie (MR), Ultraschall (US) und Tomosynthesis einzulesen und anzuzeigen.

Pathologie- und Mammographiesysteme sind vielfach zudem in eine komplexe Datenverarbeitungsumgebung einer medizinischen oder klinischen Einrichtung eingebaut und müssen mit weiteren Komponenten einer solchen Umgebung, wie beispielsweise einem "Hospital Information System" (HIS), einem "Radiology Information System" (RIS) oder einem "Picture Archiving and Communication System" (PACS) Daten austauschen.

Problematisch an heutigen Pathologie- und Mammographiesystemen ist insbesondere eine häufig vergleichsweise schlechte Ladeperformance der anzuzeigenden Bilder, d.h. einer als störend lang empfundenen Ladezeit, eine schlechte Pan&Zoom-Performance der Bilddaten und eine fehlende Unterstützung für das Einladen und Versenden von Bilddaten über das Internet.

Probleme mit der Ladeperformance entstehen insbesondere dadurch, dass die Bildaten - insbesondere im Fall der in der Pathologie gebräuchlichen WSI-Bilder - einen sehr großen Speicherplatzbedarf (im Bereich von typischerweise einigen Gigabyte) aufweisen, oder dass - insbesondere im Fall der Mammographie - sehr viele Bilder in schneller Zeit (z.B. bis zu hundert Fälle pro Stunde) geladen werden sollen.

Als "Pan&Zoom"-Funktionalität bezeichnet man die Möglichkeit, den angezeigten Bildbereich der - auf dem Bildschirm üblicherweise nur ausschnittweise darstellbaren - Bilddaten verschieben zu können ("Pan"-Funktion), um vorher verdeckte Bildbereiche sichtbar zu machen, sowie die Möglichkeit, den angezeigten Bildbereich in Relation zu der Gesamtfläche des Bildes reversibel vergrößern und verkleinern zu können ("Zoom"-Funktion). Die Pan&Zoom-Funktionalität wird insbesondere in der Pathologie aufgrund der extremen Größe und des hohen Detailreichtums der hier untersuchten Bilddaten verwendet. Die Bilder einer Studie werden hierbei zunächst in niedriger Auflösung, und somit mit einem großen sichtbaren Bildbereich angezeigt. Interessierende Stellen eines solchen Bildes werden dann vom Arzt "gepant", d.h. in das Zentrum des Anzeigerahmens gezogen, und anschließend mittels der Zoom-Funktion stark vergrößert. Probleme mit der Pan&Zoom-Performance liegen wiederum häufig in der immensen Größe der heute verwendeten Bilddatensätze begründet, zumal die Navigation in solchen großen Datensätzen extrem viel Rechenleistung erfordert.

Im Hinblick auf die Möglichkeit, Bilder über das Internet einzulesen, oder zu versenden, tritt häufig das Problem auf, dass die - üblicherweise in dem sogenannten DICOM-Format vorliegenden - Bilddaten die Internet-Firewall, die die Datenverarbeitungsstruktur einer medizinischen oder klinischen Einrichtung üblicherweise nach außen hin abgrenzen, in der Regel ohne besondere Vorkehrungen nicht durchqueren können. Der Bedarf, medizinische Bilddaten über das Internet austauschen zu können, nimmt aber durch die stetig steigende Spezialisierung und Globalisierung medizinischer Dienstleistungen zu. Insbesondere ist vergleichsweise häufig die Meinung externer Spezialisten einzuholen, die sich mitunter auf anderen Kontinenten befinden können. Hinzu kommt ein genereller Wunsch im medizinischen Bereich, die klinischen Fälle überall (zumindest) anschauen zu können, zum Beispiel auf jedem Rechner in einer Klinik, sowie auf mobilen Datenverarbeitungsgeräten wie Notebooks oder sogenannten Smartphones.

Ein weiterer Nachteil heutiger Pathologie- und Mammographiesysteme ist, dass die Anschaffungskosten für ein modernes System die Budgets kleiner und mittlerer Krankenhäuser häufig übersteigt. Dies führt dazu, dass sich nur große klinische Einrichtungen diese Investition leisten können. Kleinere klinische Institutionen müssen häufig auf moderne digitale Pathologie- oder Mammographiesysteme aus Kostengründen verzichten.

Ferner verursacht die heute übliche Einrichtung von separaten Pathologie- oder Mammographiesystemen für jede klinische Einrichtung durch die Vielzahl vorhandener und zu betreibender Systeme insgesamt einen vergleichsweise hohen Strom- und Ressourcenverbrauch, der dem Wunsch nach einer ökologischen vertretbaren Informationstechnologie (Green IT) kaum gerecht werden kann.

Der Erfindung liegt die Aufgabe zugrunde, ein Verfahren zur Anzeige von medizinischen Bilddaten, insbesondere im Bereich der Pathologie oder im Bereich der Mammographie, anzugeben, das im Hinblick auf die vorstehend beschriebenen Probleme verbessert ist. Der Erfindung liegt des Weiteren die Aufgabe zugrunde, eine für die Durchführung dieses Verfahrens besonders geeignete Einrichtung anzugeben.

Bezüglich des Verfahrens wird die Aufgabe erfindungsgemäß gelöst durch die Merkmale des Anspruchs 1. Bezüglich der zugehörigen Einrichtung wird die Aufgabe erfindungsgemäß gelöst durch die Merkmale des Patentanspruchs 10. Vorteilhafte Ausführungen und Weiterbildungen der Erfindung sind in den jeweiligen Unteransprüchen dargelegt.

Die erfindungsgemäße Einrichtung umfasst ein zentrales Datencenter sowie mindestens einen Client. Bei dem Datencenter handelt es sich um eine Datenverarbeitungsanlage, die beispielsweise aus einem oder mehreren Servern mit einem darauf installierten Softwaresystem gebildet ist. Bei dem Client handelt es sich um einen gewöhnlichen Arbeitsrechner oder Kleinrechner, insbesondere um einen Desktop-PC, ein Notebook, Tablet-Computer, ein PDA oder ein Smartphone. Das Datencenter einerseits und die Clients andererseits werden dabei typischerweise von verschiedenen Unternehmen oder Organisationen betrieben. Insbesondere sind die Clients Bestandteil der internen Datenverarbeitungs-Struktur (IT-Struktur) von Arztpraxen und Kliniken, wobei eine Vielzahl solcher medizinischer Betriebe auf das von einem unabhängigen Anbieter betriebene Datencenter zugreifen. Damit verbunden stammen die Hard- und Software des Datencenters einerseits, und die Hard- und Software der Clients andererseits häufig von verschiedenen Herstellern.

Das Datencenter ist mit dem oder jedem Client über eine schnurgebundene oder schnurlose Datenkommunikationsverbindung, insbesondere ein LAN oder W-LAN und/oder über das Internet verbunden. Auf dem Client ist zumindest ein Web-Browser installiert.

Im Zuge des in und mittels der vorstehend beschriebenen Einrichtung ausgeführten Verfahrens wird zunächst aus einem medizinischen Bilddatensatz (nachfolgend als "Eingangsbilddatensatz" bezeichnet), der im DICOM-Format oder einem vergleichbaren Datenformat für medizinische Bilddaten vorliegt, eine Hierarchiegruppe von pyramidentransformierten Bildkacheln erzeugt.

Als "Pyramidentransformation" wird dabei ein Bildbearbeitungsverfahren bezeichnet, bei dem aus dem als Primärbild herangezogenen Eingangsbilddatensatz mehrere Sekundärbilder erzeugt werden, die im Wesentlichen die gleiche Bildgrundinformation beinhalten wie der Eingangsbilddatensatz, die diese Bildgrundinformation aber jeweils in unterschiedlicher Bildauflösung wiedergeben. Da mit abnehmender Bildauflösung die Pixelanzahl und somit die Größe der Sekundärbilder abnimmt, bilden diese Sekundärbilder, wenn man sie gedanklich der Größe nach übereinander stapelt, eine sogenannte Bildpyramide, die namensgebend für dieses Bildbearbeitungsverfahren ist. Bei der erfindungsgemäßen Pyramidentransformation werden die die Bildpyramide bildenden Sekundärbilder nicht in einem gemeinsamen Bilddatensatz zusammengefasst. Vielmehr werden die einzelnen Sekundärbilder - ggf. mit Ausnahme der Pyramidenspitze, d.h. des Sekundärbildes mit der geringsten Auflösung - jeweils wiederum in kleinere Bilddatensätze (im folgenden "Bildkacheln" genannt) aufgeteilt. Die aus dem Eingangsbilddatensatz abgeleitete Hierarchiegruppe von Bildkacheln umfasst daher mehrere Untergruppen, deren Bildkacheln jeweils ein Sekundärbild der Bildpyramide bilden. Eine vereinfachte dreistufige Hierarchiegruppe von Bildkacheln umfasst beispielsweise
- 16 Bildkacheln, die dem untersten Sekundärbild der Bildpyramide entsprechen, und die jeweils ein Sechszehntel der Bildinformation des Eingangsbilddatensatzes mit einer diesem entsprechenden Auflösung abbilden,
- 4 Bildkacheln, die einem mittleren Sekundärbild der Bildpyramide entsprechen, und die jeweils ein Viertel der Bildinformation des Eingangsbilddatensatzes mit halber Auflösung abbilden, sowie
- eine Bildkachel, die das oberste Sekundärbild der Bildpyramide bildet, und die die gesamte Bildinformation des Eingangsbilddatensatzes mit einem Viertel der Auflösung des Eingangsbilddatensatzes abbildet.

Die erzeugten Bildkacheln werden in einem Bildkachelspeicher des Datencenters hinterlegt.

In einem weiteren Verfahrensschritt wird jeder Bildkachel ein sogenannter Uniform Ressource Locator (URL) zugewiesen, wobei diese URL unmittelbar oder mittelbar eine Information über die Bildauflösung der Bildkachel sowie über den von dieser Bildkachel abgedeckten Bildausschnitt des Eingangsdatensatzes enthält. Diese Information wird insbesondere durch eine Namenskonvention codiert in URLs und Dateinamen der Bildkacheln wiedergeben.

Clientseitig wird zu einem späteren Zeitpunkt mittels des dort installierten Web-Browsers eine Internet-Applikation ausgeführt, mittels der - automatisch oder in Interaktion mit einem Client-Nutzer - ein anzuzeigender Bildbereich des Eingangsbilddatensatzes sowie eine zugehörige Bildauflösung gewählt werden. Der Bildbereich und die Bildauflösung ändern sich hierbei dynamisch, insbesondere infolge von Benutzerinteraktionen.

Durch die Internet-Applikation wird nun die URL der oder jeder Bildkachel ermittelt und aufgerufen, die hinsichtlich ihrer Bildauflösung und ihres Bildausschnittes mit der gewählten Bildauflösung und dem gewählten Bildbereich - identisch oder zumindest bestmöglich - übereinstimmen. Sofern der gewählte Bildbereich mit dem Bildausschnitt mehrerer Bildkacheln überlappt, werden die URLs aller dieser Bildkacheln bestimmt und aufgerufen. Der Aufruf auf die oder jede URL wird dabei insbesondere dynamisch durch die Internet-Applikation erzeugt.

Unter Zugriff auf die oder jede bestimmte URL wird durch die Internet-Applikation die oder jede zugehörige Bildkachel aus dem Bildkachelspeicher des Datencenters in den Client geladen und dort - zumindest soweit der anzuzeigende Bildbereich dem Bildausschnitt der jeweiligen Bildkachel entspricht - angezeigt.

Durch die Aufteilung des - regelmäßig sehr großen - Eingangsbilddatensatzes in eine Vielzahl von vergleichsweise kleinen Bildkacheln wird eine wesentliche Verbesserung der Ladeperformance erzielt, zumal nie der gesamte Eingangsbilddatensatz geladen werden muss (und geladen wird), sondern stets nur diejenigen Bilddaten, die gerade angezeigt werden sollen. Infolge der Pyramidentransformation, und der daraus resultierenden Bereitstellung der Bildinformation in mehreren unterschiedlichen Auflösungstufen können dabei große Bildbereiche (mit geringer Bildauflösung) vergleichbar schnell geladen werden wie kleine Bildbereiche (mit hoher Bildauflösung).

Um einen schnellen Bildaufbau sicherzustellen, werden vorzugsweise zunächst Bildkacheln mit geringer Auflösung geladen und angezeigt. Anschließend werden Bildkacheln mit sukzessive steigender Auflösung dynamisch nachgeladen, bis der gewählte Auflösungsgrad erreicht ist.

In bevorzugter Ausführung der Erfindung ist die Internet-Applikation als sogenannte Rich-Internet-Application (RIA) implementiert. Das bedeutet, dass die gesamte Funktionalität, die zum Einlesen der Bildkacheln inklusive des dynamischen Nachladens benötigt wird, ausschließlich in der Internet-Applikation implementiert ist und somit nur clientseitig ausgeführt wird. Mit anderen Worten sind hierfür im Datencenter keine Serverdienste vorgesehen. Vielmehr reduziert sich die Interaktion zwischen den Clients und dem Datencenter beim Laden der Bildkacheln auf Zugriffe auf das Dateisystem des Datencenters. Das Datencenter stellt somit diesbezüglich quasi ein weltweit zugängliches Speichermedium dar, das für das Einlesung und Nachladen der Bildkacheln selbst keinen Rechenaufwand leistet. Dies ermöglicht eine nahezu unendliche Skalierbarkeit des Verfahrens und der zugehörigen Einrichtung, da im Datencenter für das Einlesung und Nachladen der Bildkacheln keine Serverkapazität vorgehalten werden muss.

Durch den Einsatz an sich gewöhnlicher Internet-Technologie, insbesondere durch den Einsatz eines Web-Browsers zum Ausführen der als Internet-Applikation implementierten Anzeige-Software sowie durch den Zugriff auf die zu ladenden Bildkacheln über URLs, wird dabei gleichzeitig die flexible Nutzbarkeit des Verfahrens unter verschiedensten clientseitig vorliegenden Bedingungen entscheidend verbessert. Insbesondere kann das Verfahren clientseitig in gleicher Weise mit Desktop-PCs unterschiedlicher Betriebssysteme wie mit PDAs und SmartPhones durchgeführt werden, ohne an diesen Geräten gegenüber der Standard-Funktionalität etwas ändern zu müssen. Insbesondere muss - außer dem in aller Regel ohnehin zur Standardausrüstung gehörenden Web-Browser - clientseitig keine Spezial-Software installiert werden. Folglich werden zur Implementierung des Verfahrens und der zugehörigen Einrichtung auf dem oder jeden Client auch keine Administrator-Rechte benötigt.

Die Internet-Applikation kann dabei lokal in einem Speicher des Clients hinterlegt sein. In bevorzugter Ausbildung wird die Internet-Applikation aber in dem Datencenter zum Herunterladen (Download) durch den oder jeden Client zur Verfügung gestellt, so dass in den Clients keinerlei spezielle Vorbereitungen oder Voreinstellungen für die Verfahrensdurchführung vorgenommen werden müssen.

Der Einsatz an sich gewöhnlicher Internet-Technologie für das Übertragen der Bilddaten erleichtert auch das Einlesen und Versenden der Bilddaten über das Internet erheblich. Insbesondere stellen herkömmliche Internet-Firewalls für diesen Datenaustausch kein Hindernis dar, ohne dass solche Firewalls in besonderer Weise konfiguriert werden müssten. Das Verfahren kann daher an jedem - mit dem Internet verbundenen - geographischen Punkt aus durchgeführt werden, ohne spezielle Vorbereitungen durchführen zu müssen.

Um die Performance des Verfahrens beim Laden der Bilddaten weiter zu verbessern, ist in bevorzugter Ausführung der Erfindung vorgesehen, dass die Bildkacheln in dem Datencenter in einem komprimierten Bilddatenformat, insbesondere JPEG oder PNG, erzeugt werden, und somit das zu übertragende Datenvolumen reduziert wird.

Um auch für Clients mit stark unterschiedlicher Rechenleistung stets die für einen befriedigenden Verfahrensablauf nötige Performance sicherzustellen, ist zweckmäßigerweise vorgesehen, dass die Internet-Applikation dazu eingerichtet ist, sich hinsichtlich der Kompression der Bilddaten und der Darstellungsgenauigkeit an die Rechenleistung des individuellen Clients, auf dem sie abläuft, zu adaptieren.

Zusätzlich oder alternativ werden durch die Internet-Applikation zweckmäßigerweise Bilddaten nach dem ersten Einlesen in einem Cache-Speicher des Clients zwischengespeichert, von wo aus sie im Falle eines zweiten und jedes weiteren Aufrufs ressourcen- und zeitsparend geladen werden. Das Zwischenspeichern der Bildkacheln wird vorzugsweise durch die Caching-Funktion des Web-Browsers vorgenommen, die bei gängigen Web-Browsern ohnehin standardmäßig vorhanden ist. Die Caching-Funktion ist insbesondere wichtig bei der Darstellung von Bewegtbild-Sequenzen (Movie-Mode), bei der gleiche Bildkacheln in häufiger Wiederholung gezeigt werden.

Wiederum zusätzlich oder alternativ ist verfahrensgemäß vorgesehen, dass das Einlesen der Bildkacheln aus dem Bildkachelspeicher durch die Internet-Applikation ausschließlich mittels asynchroner Kommunikation vorgenommen wird, so dass ein Ladevorgang zu einem beliebigen Zeitpunkt gestartet werden kann, auch wenn der vorausgehende Ladevorgang noch nicht abgeschlossen ist.

Um eine einfache und schnelle Auffindbarkeit von relevanten Bilddatensätzen sicherzustellen, wird verfahrensgemäß in dem Datencenter zu dem oder jedem Eingangsbilddatensatz Indexinformation erzeugt und hinterlegt, die den Speicherort der zugehörigen Bildkacheln identifiziert. Die Indexinformation enthält zudem weitere mit dem Eingangsbilddatensatz verknüpfte (also dem Eingangsbilddatensatz zugehörige) Angaben. Hierzu gehören insbesondere Angaben, die die Entstehungsbedingungen und die Bearbeitungsgeschichte der Eingangsbilddaten charakterisieren, z.B. Angaben zu der bilderzeugenden Modalität, dem Patienten, dem Befundungsstatus und/oder dem zugeordneten Arzt.

In bevorzugter Ausführung dient das Verfahren nicht lediglich zum bloßen Anzeigen der medizinischen Bilddaten, sondern unterstützt zusätzlich auch die Auswertung (Befundung) dieser Bilddaten. Hierzu wird in einer zweckmäßigen Variante der Erfindung durch die Internet-Applikation über eine Nutzerschnittstelle mindestens eine Bildauswertungs-Methode zur Verfügung gestellt. In diesem Sinne sind im Rahmen der Internet-Applikation insbesondere
- mindestens eine Methode zur Vermessung der Bildinformation, z.B. eine Methode zur Erzeugung einer Distanzlinie,
- mindestens eine Methode zur Markierung von Bildbereichen,
- mindestens eine Methode zur Beschreibung von Befunden, und/oder
- mindestens eine Methode zur Beschriftung der Bildinformation
   implementiert. Befundungsergebnisse, die von einem Client-Nutzer markiert und/oder eingegeben worden sind, werden vorzugsweise durch die Internet-Applikation gesammelt und in einem Befundspeicher des Datencenters hinterlegt.

Zweckmäßigerweise werden durch die Internet-Applikation zusätzlich oder alternativ über eine Nutzerschnittstelle Navigationsmittel für eine Pan&Zoom-Funktion zur Verfügung gestellt, mittels derer ein Client-Nutzer den anzuzeigenden Bildbereich reversibel verschieben und vergrößern oder verkleinern kann.

Das Datencenter der erfindungsgemäßen Einrichtung ist in bevorzugter Ausführung gegliedert in ein Speicher-Service-Modul sowie in ein Backend-Service-Modul. Bei dem Speicher-Service-Modul handelt es sich bevorzugt um eine Filesystem-Struktur, in der die Bildkacheln und ggf. die Download-Datei der Internet-Applikation internet-weit verfügbar gespeichert sind. Bei dem Backend-Service-Modul handelt es sich bevorzugt um ein Softwaremodul, das transparent, also für den Client unsichtbar, im Hintergrund des Datencenters abläuft. Das Backend-Service-Modul ist dabei programm- und/ oder schaltungstechnisch dazu eingerichtet, aus dem Eingangsbilddatensatz die zugehörige Hierarchiegruppe von Bildkacheln zu erzeugen, und diese Bildkacheln in dem Bildkachelspeicher des Speicher-Service-Moduls zu hinterlegen. Das Backend-Service-Modul ist weiterhin dazu eingerichtet, jeder Bildkachel eine URL zuzuweisen, die eine Information über die Bildauflösung der Bildkachel und den von Ihr abgedeckten Bildausschnitt enthält.

Das Backend-Service-Modul ist vorzugsweise weiterhin programmtechnisch dazu eingerichtet, zu dem oder jedem Eingangsbilddatensatz Indexinformation zu erzeugen und zu hinterlegen, die den Speicherort der zugehörigen Bildkacheln sowie mit dem Bilddatensatz verknüpfte Angaben, insbesondere zu der Modalität, dem Patienten, dem Befundungsstatus und/oder dem zugeordneten Arzt enthält.

Die Eingangsdatensätze werden der Einrichtung beispielsweise aus einer medizinischen Modalität übermittelt, und zwar entweder direkt oder über ein weiteres computergestütztes System, beispielsweise ein PACS oder RIS. Um Ressourcen des Datencenters sparen zu können und gleichzeitig eine gute Performance der Kommunikation zwischen dem Datencenter und den Clients sicherzustellen, ist das Backend-Service-Modul dabei vorzugsweise dazu eingerichtet, die Extraktion von Bildkacheln aus Eingangsdatensätzen zeitlich getrennt von der Anzeige und Befundung der Bilddaten durchzuführen. Beispielsweise werden die Bildkacheln zu neuen Eingangsbilddatensätze mit erhöhter Priorität während der Nachtzeiten erzeugt, in denen das Datencenter keiner oder nur einer geringen Anfragelast durch die damit verbundenen Clients ausgesetzt ist.

Das vorstehend beschriebene Verfahren oder die zugehörige Einrichtung sind insbesondere für eine Verwendung zur Anzeige von Pathologie-Bilddaten oder Mammographie-Bilddaten vorgesehen.

Nachfolgend werden Ausführungsbeispiele der Erfindung anhand einer Zeichnung näher erläutert. Darin zeigen:
- FIG 1: in einem schematischen Blockschaltbild eine Einrichtung zur Anzeige von medizinischen Bilddaten sowie zur Unterstützung der Auswertung dieser Bilddaten, mit einem Datencenter und einer Anzahl von datenkommunikationstechnisch mit diesem verbundenen Clients,
- FIG 2: in einem schematischen Blockschaltbild ein Datencenter der Einrichtung gemäß FIG 1,
- FIG 3: in schematischer Darstellung eine Bildpyramide von in dem Datencenter hinterlegten Bildkacheln, die die Bildinformation eines medizinischen Bildatensatzes wiedergeben, und
- FIG 4: in einem Blockschaltbild ein Deployment-Schema der Einrichtung in deren Betrieb.

Einander entsprechende Teile, Größen und Strukturen sind in den Figuren stets mit gleichen Bezugszeichen versehen.

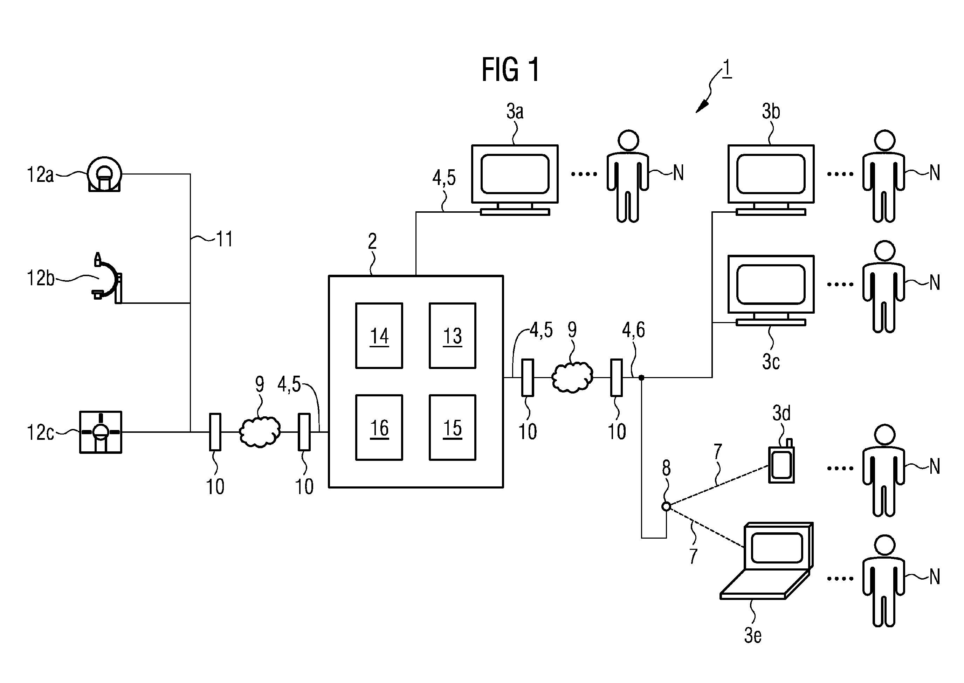

Die in FIG 1 dargestellte Einrichtung 1 umfasst ein Datencenter 2 sowie eine Anzahl von - hier vereinfacht fünf - Clients 3a-3e, die über ein Datenkommunikationsnetz 4 mit dem Datencenter 2 verbunden sind.

Bei dem Datencenter 2 handelt es sich um eine Serverfarm mit einem darauf implementierten Softwaresystem, das den funktionellen Kern der Einrichtung 1 bildet.

Bei den Clients 3a-3e kann es sich um beliebige, insbesondere auch unterschiedliche Arbeits- oder Kleinrechner handeln. Zur Verdeutlichung der hier möglichen Bandbreite sind die Clients 3a-3c beispielhaft als gewöhnliche Desktop-Personal-Computer (PC), der Client 3d als Personal Digital Assistant (PDA) oder Smart-Phone, und der Client 3d als mobiler PC (Notebook) dargestellt. Auf den Clients 3a-3e ist keine spezielle Softwareausstattung vorgesehen. Um im Rahmen der Einrichtung 1 verwendbar zu sein, muss auf den Clients 3a-3e außer einem üblichen Betriebssystem, das den Anschluss des Clients 3a-3e an das Datenkommunikationsnetz 4 ermöglicht, lediglich ein üblicher Web-Browser installiert sein.

Zu jedem Client 3a-3e ist in FIG 1 ein mit diesem interagierender Nutzer N dargestellt. Bei den Nutzern N handelt es sich insbesondere um Ärzte, die die über die Clients 3a-3e anzuzeigenden Bilder befunden.

Das Datenkommunikationsnetz 4 umfasst im dargestellten Beispiel ein sogenanntes Local Area Network (LAN) 5, über das das Datencenter 2 mit dem Client 3a direkt verbunden ist. Bestandteile des Datenkommunikationsnetzes 4 sind darüber hinaus
- ein weiteres LAN 6 (z.B. einer Klinik), in das die Clients 3b und 3c eingebunden sind,
- ein Wireless Local Area Network (WLAN) 7, das von dem LAN 6 über einen Router 8 abzweigt, und in das die Clients 3d und 3e eingebunden sind, sowie
- das Internet 9, über das das LAN 5 und das LAN 6 verbunden sind.

Die LANs 5 und 6 sind zum Internet 9 hin jeweils durch eine Firewall 10 abgeschottet.

Das Datencenter 2 ist ferner über das LAN 5 und das Internet 9 mit einem weiteren LAN 11 (z.B. einer weiteren Klinik) verbunden, in das verschiedene bildgebende Modalitäten 12a-12c eingebunden sind. Beispielsweise handelt es sich bei der Modalität 12a um einen Computertomographen, bei der Modalität 12b um ein Röntgen-C-Bogen-Gerät und bei der Modalität 12c um einen Magnetresonanztomographen.

Das in dem Datencenter 2 implementierte Softwaresystem ist grob in vier Einheiten oder Module gegliedert, nämlich in
- ein Speicher-Service-Modul 13,
- ein Frontend-Service-Modul 14,
- ein Backend-Service-Modul 15, und
- ein Datenbank-Service-Modul 16.

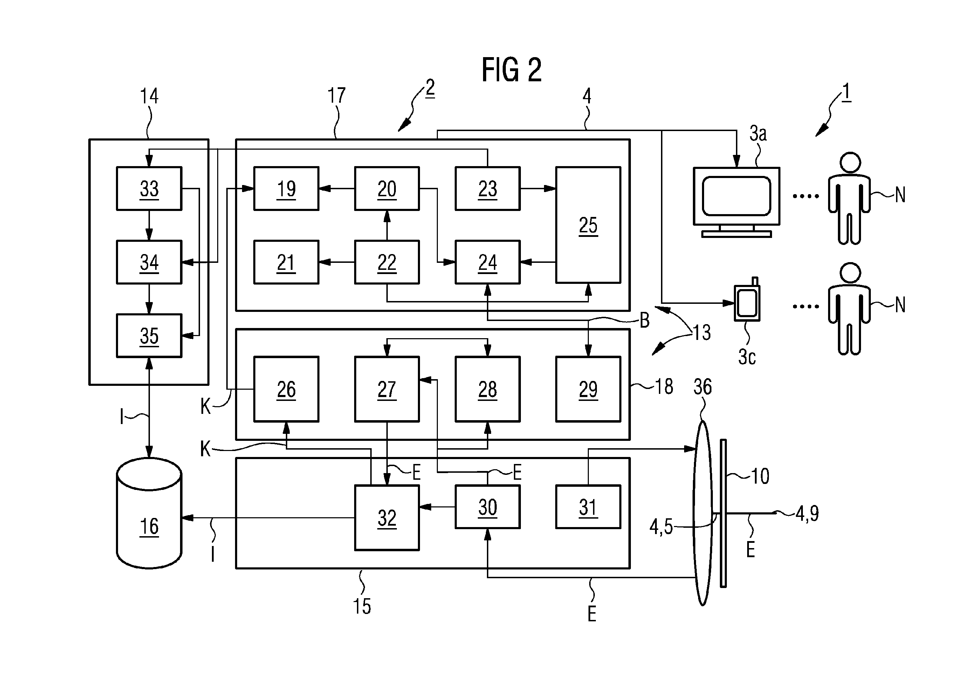

Die im Normalbetrieb der Einrichtung 1 wichtigste Einheit des Datencenters 2 ist das Speicher-Service-Modul 13. Dieses Speicher-Service-Modul 13 stellt das Dateisystem des Datencenters 2 dar. Wie aus FIG 2 hervorgeht, enthält das Speicher-Service-Modul 13 zum Einen den ausführbaren Code (Download-Datei) einer Internet-Applikation 17, d.h. einer Softwareanwendung, die in dem Web-Browser eines jeden der Clients 3a-3e ausführbar ist, und die die im Normalbetrieb der Einrichtung 1 benötigte Funktionalität (Business Logik) bündelt. Zum Anderen enthält das Speicher-Service-Modul 13 einen Kontextspeicher 18, in dem die von der Internet-Applikation 17 benötigten und erzeugten Daten gespeichert werden.

Die Internet-Applikation 17, die in dem Datencenter 2 nicht selbst ausgeführt, sondern nur zum Download auf die Clients 3a-3e bereitgestellt wird, ist eine "Rich-Internet-Application" (RIA), die als funktionale Einheiten oder Module enthält:
- Ein Kachellademodul 19, das dazu eingerichtet ist, vorbereitete und hinterlegte Hierarchien von Bildkacheln K medizinischer Bilddaten aus dem Datencenter 2 in den jeweiligen Client 3a-3e zu laden.
- Ein Szenenrendermodul 20, das dazu eingerichtet ist, die geladenen Bildkacheln K zu einer anzeigbaren Gesamtbilddarstellung (Szene) zusammenzufügen.
- Ein Methodenmodul 21, das Methoden zur Auswertung der angezeigten Bilddaten implementiert, nämlich insbesondere
   o eine Methode zum Ändern des Layouts der angezeigten Bilddaten (Der Begriff "Layout" bezeichnet hierbei eine Konfigurations- oder Vorlagen-Datei, in der insbesondere die Anzahl, Art, Größe und/oder Bildschirmanordnung der anzuzeigenden Bilder festgelegt ist),
   o eine Methode zur Erzeugung von Distanzlinien, die aus den Bilddaten, beispielsweise zur Vermessung eines Tumors, den realen Abstand zwischen zwei durch die Distanzlinie verbundenen Bildpunkten berechnet,
   o eine Methode zur Eingabe von medizinischen Befunden B,
   o eine Methode zum Vergrößern und Verkleinern eines angezeigten Bildbereichs eines medizinischen Bildes,
   o eine Methode zum Verschieben des angezeigten Bildbereichs, sowie
   o eine Methode zum Markieren und Beschriften der Bilddaten,
   o ferner eine Methode zum Senden von DICOM-Daten, für die das Methodenmodul 21 auf ein (nachfolgend beschriebenes) DICOM-Sendemodul 31 des Backend-Service-Moduls 15 zugreift,
- Ein als Layout-Manager 22 bezeichnetes Softwaremodul, das dazu eingerichtet ist, Segmente der Internet-Applikation 17, deren Layout von dem oder jedem Nutzer N ausgewählt werden kann, und in die die Bildkacheln K geladen werden können, zu verwalten.
- Ein als Worklistmanager 23 bezeichnetes Softwaremodul, das dazu eingerichtet ist, dem Nutzer N sogenannte Worklists (d.h. Listen mit zu erledigenden Aufgaben) zu präsentieren.
- Ein als Befundmanager 24 bezeichnetes Softwaremodul, das dazu eingerichtet ist, Befunde B, die der Nutzer N bei der Auswertung der angezeigten Bilddaten eingibt, zu verwalten.
- (Optional) ein als Double-Reading-Manager 25 bezeichnetes Softwaremodul, das dazu eingerichtet ist, ein Double-Reading-Verfahren zur Verifizierung der Befunde B zu veranlassen.

Der Kontextspeicher 18 enthält als Einheiten oder Module
- einen Bildkachelspeicher 26, der dazu eingerichtet ist, Bildkachelhierarchen von medizinischen Bildern zu speichern,
- einen DICOM-Bildspeicher 27, der dazu eingerichtet ist, DICOM-Bilddaten, die den vorstehend genannten Bildkacheln K zugrundeliegen, zu speichern,
- (optional) ein Vorverarbeitungsmodul 28, das dazu eingerichtet ist, anwendungsspezifische Vorverarbeitungsschritte an den Bilddaten vorzunehmen (im Anwendungsfall der Mammographie besteht ein solcher Vorverarbeitungsschritt beispielsweise darin, die Schichten von Bildern der linken und der rechten Brust aneinander anzugleichen) sowie
- einen Befundspeicher 29 zum Speichern der Befunde B.

Das Speicher-Service-Modul 13 stellt die in ihm gespeicherten Daten internet-weit zum Abruf (Download) bereit.

Das Backup-Service-Modul 15 dient vorrangig dazu, zugeführte DICOM-Daten zu empfangen und daraus eine zugehörige Bildkachelhierarchie zu erzeugen. Es besteht im Wesentlichen aus drei Komponenten, nämlich
- einem DICOM-Empfangsmodul 30, das dazu eingerichtet ist, DICOM-Daten von den Modalitäten 12a-12c oder einer anderen Einrichtung, z.B. einem RIS, zu empfangen,
- einem DICOM-Sendemodul 31, das dazu eingerichtet ist, DICOM-Daten an andere Einrichtungen zu senden, sowie
- einem (Dicom/JPEG-)Konverter 32, der dazu eingerichtet ist, die eingehenden DICOM-Bilddatensätze in die genannten Bildkacheln K zu transformieren und die Bildkacheln K in dem Bildkachelspeicher 26 abzuspeichern. Der Konverter 32 dient weiterhin dazu, zu jedem eingehenden DICOM-Bilddatensatz E Angaben zum Speicherort, zur Modalität, zum Patienten, zum Befundungsstatus und zu dem betreuenden Arzt als Indexinformaton I zu erfassen.

Das Datenbank-Service-Modul 16 enthält eine Datenbank, in der die von dem Dicom/JPEG-Konverter 32 erfasste Indexinformation I hinterlegt ist.

Das Frontend-Service-Modul 14 enthält Dienste, die im Datencenter 2 ausgeführt werden, und die zwischen der - clientseitig ausgeführten - Internet-Applikation 17 einerseits und dem Datenbank-Service-Modul 16 andererseits vermitteln. Das Frontend-Service-Modul 14 besteht im Wesentlichen aus drei Komponenten, nämlich
- einem als Datenbank-Worklist-Manager 33 bezeichneten Softwaremodul, das dazu eingerichtet ist, Informationen über die vorstehend erwähnten Worklists in dem Datenbank-Service-Modul 16 abzuspeichern und aus diesem herauszulesen,
- ein als Datenbank-Bild-Manager 34 bezeichnetes Softwaremodul, das dazu eingerichtet ist, Informationen über die DICOM-Bilddaten und die zugehörigen Bildkacheln K in dem Datenbank-Service-Modul 16 abzuspeichern und aus der Datenbank herauszulesen, und
- ein als Datenbank-Zugriffs-Manager 35 bezeichnetes Softwaremodul, das dazu eingerichtet ist, die Zugriffe auf das Datenbank-Service-Modul 16 semantikfrei und zentral zu steuern und die Datenbank somit zu kapseln.

Im Betrieb der Einrichtung 1 wird ein zum Beispiel von einer der Modalitäten 12a-12c eingehender (Eingangs-)Bilddatensatz E zunächst von dem Backend-Service-Modul 15 verwaltet. Um eine problemlose Übermittlung des Bilddatensatzes E über das Internet 9 sicherzustellen, wird der - ursprünglich im DICOM-Format erzeugte - Bilddatensatz E zunächst in ein dem HTTP-Protokoll entsprechendes Format transformiert und in dieser Form an das Datencenter 2 übermittelt. Durch ein als Port-Mediator 36 bezeichnetes Softwaremodul, das der Firewall 10 des Datencenters 2 nachgeschaltet ist, wird hierbei der Bilddatensatz E von dem HTTP-Format in das DICOM-Format rücktransformiert. Der solchermaßen rücktransformierte Bilddatensatz E wird innerhalb des Backend-Service-Moduls 15 zunächst dem DICOM-Empfangsmodul 30 zugeführt.

Das DICOM-Empfangsmodul 30 speichert den Eingangsdatensatz E in dem DICOM-Bildspeicher 27 ab. Außerdem aktiviert das DICOM-Empfangsmodul 30 das Vorverarbeitungsmodul 28 (sofern dieses vorgesehen ist), das an dem gespeicherten Bilddatensatz E die ggf. gewünschten Vorverarbeitungsschritte vornimmt.

Schließlich gibt das DICOM-Empfangsmodul 30 einen Auftrag an den DICOM-JPEG-Konverter 32.

Der Konverter 32 lädt im direkten Anschluss oder zu einem späteren Zeitpunkt den Bilddatensatz E aus dem DICOM-Bildspeicher 27 und pyramidentransformiert den geladenen Bilddatensatz E in eine Bildpyramide, d.h. Hierarchiegruppe von Bildkacheln K unterschiedlicher Bildauflösung.

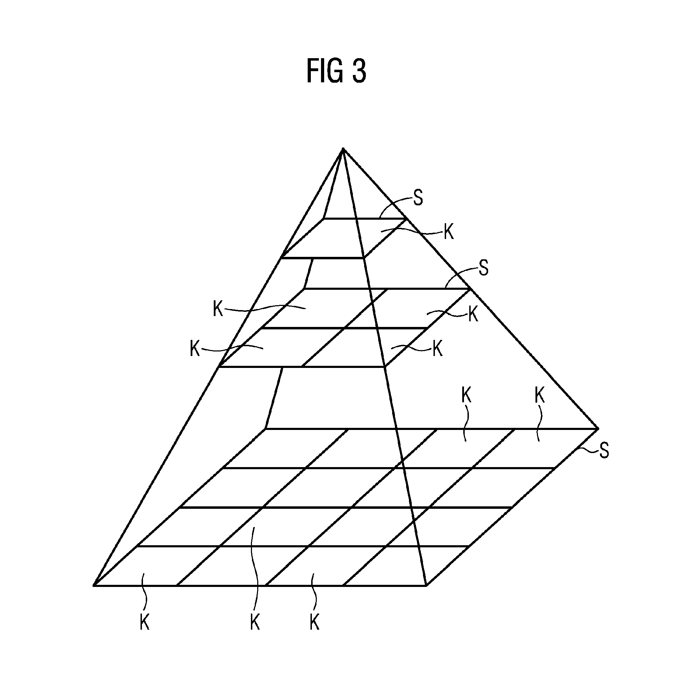

Die - in FIG 3 in stark vereinfachter Form dargestellte - Bildpyramide enthält im Beispiel in drei Hierachieebenen je ein aus dem Bilddatensatz E abgeleitetes Sekundärbild S, wobei sich die Bildauflösung der Sekundärbilder S der mittleren und oberen Ebene im Vergleich zu dem nächstunteren jeweils Sekundärbild S jeweils halbiert. Durch die derselben Hierarchieebene der Bildpyramide zugeordneten Bildkacheln K wird das jeweils zugehörige Sekundärbild S in eine Anzahl von Ausschnitten aufgeteilt. Die Zahl der Bildkacheln K nimmt hierbei nach oben hin von Hierarchieebene zu Hierarchieebene um einen Faktor 1/4 ab. Auf diese Weise haben alle Bildkacheln einer Hierarchiegruppe die gleiche Größe (Pixelzahl)und somit auch einen vergleichbaren Speicherplatzbedarf.

Im dargestellten Beispiel hat das unterste Sekundärbild S der Bildpyramide die gleiche Bildauflösung wie der Eingangsbilddatensatz E und wird durch den Konverter 32 in sechszehn gleich große Bildkacheln K aufgeteilt. Das mittlere Sekundärbild S hat im Vergleich hierzu die halbe Bildauflösung und wird aus vier Bildkacheln K zusammengesetzt. Die Pyramidenspitze, d.h. das oberste Sekundärbild S der Bildpyramide hat 1/4 der Bildauflösung des Bilddatensatzes E und besteht aus einer einzigen Bildkachel K.

Die Bildkacheln K werden von dem Konverter 32 speicherplatzsparend in dem komprimierten JPEG-Format erzeugt und in dem Bildkachelspeicher 26 abgelegt. Der Konverter 32 ordnet jeder Bildkachel K hierbei eine URL zu, die die Bildauflösung der Bildkachel K und den von Ihr abgedeckten Bildausschnitt des zugehörigen Sekundärbildes S direkt oder indirekt wiedergibt.

Eine solche URL ist insbesondere nach der Namenskonvention
http://<Web-Adresse des Datencenters 2>/...
.../<Speicherpfad des Speicher-Service-Modul 13>/...
.../<Identitätsnummer der Studie>/...
.../<Auflösungsebene der Bildpyramide>/...
...<Position der Bildkachel K>.jpg
gebildet. In einem beispielhaften URL-Aufruf
http://www.datencenterxyz.com/studies/...
.../1.0.2,1.00/10/0_0,jpg
stehen nach der obigen Namenskonvention
- "www.datencenterxyz.com" für die (fiktive) Web-Adresse des Datencenters 2,
- "studies" für den Speicherpfad des Speicher-Service-Modul 13,
- "1.0.2.1.00" für die Identitätsnummer der Studie,
- "10" für die Auflösungsebene der Bildpyramide, und
- "0_0" für die Position der Bildkachel K.

Durch die Angabe der Auflösungsebene ist hierbei implizit gleichzeitig die Größe der dieser Ebene zugeordneten Bildkacheln K festgelegt. Beispielsweise haben die Bildkacheln K der Auflösungsebene "10" eine Größe von jeweils 248 x 248 = 1024 Bildpunkten. Allerdings können mehrere oder gar alle Auflösungsebenen Bildkacheln K der gleichen Größe aufweisen.

Die Positionsangabe "0_0" bezeichnet nach der Namenskonvention <x-Richtung_y-Richtung> die erste Bildkachel K in x-Richtung sowie die erste Bildkachel K in y-Richtung, inbesondere also diejenige Bildkachel K, die die linke obere Ecke eines größeren Bildes bildet.

Der obige URL-Aufruf führt folglich zum Download der ersten Bildkachel K in x- und y-Richtung des unter dem Studiennamen 1.0.2.1.00 hinterlegten Bildes in der zehnten Auflösungsebene.

Ferner indexiert der Konverter 32 den Bilddatensatz E in Hinblick auf den Speicherort des Bilddatensatzes E und der aus diesem abgeleiteten Bildkacheln K, sowie in Hinblick auf Modalität, Patient, Befundungsstatus und Zuordnung des zuständigen Arztes, und legt diese Indexinformation I in dem Datenbank-Service-Modul 16 ab.

Um die in dem Datencenter 2 gespeicherten Bilddaten anzusehen und auszuwerten, startet ein Nutzer N zunächst den Web-Browser des ihm - im Zusammenhang mit FIG 2 angenommenerweise - zur Verfügung stehenden Clients 3a oder 3d und lädt über eine vorgegebene URL die in dem Speicher-Service-Modul 13 des Datencenters 2 vorgehaltene Internet-Applikation 17 in den Client 3a bzw. 3d.

Im Rahmen der in dem Web-Browser ablaufenden Internet-Applikation 17 wird zunächst der Worklist-Manager 23 ausgeführt, der dem Nutzer N eine Anzahl von zu bearbeitenden Aufgaben zur Auswahl anzeigt. Der clientseitig laufende Worklist-Manager 23 erhält die Information über zu bearbeitende Aufgaben durch Kommunikation mit dem Datenbank-Worklist-Manager 33 des im Datencenter 2 ablaufenden Frontend-Service-Moduls 14, das diese Information wiederum durch - über den Datenbank-Zugriffsmanager 35 vermittelten - Zugriff auf das Datenbank-Service-Modul 16 ermittelt.

Wenn der Nutzer N eine der angezeigten Aufgaben auswählt, holt der Worklist-Manager 23 über den Datenbank-Worklist-Manager 33 und den Datenbank-Bildmanager 34 Information über den Speicherort der mit dieser Aufgabe verknüpften Bilddaten ein. Der Datenbank-Bildmanager 34 ermittelt diese Information ebenfalls, indem er über den Datenbank-Zugriffsmanager 35 auf das Datenbank-Service-Modul 16 zugreift. Mit den von dem Datenbank-Bildmanager 34 gelieferten Daten erzeugt das Kachellademodul 19 nun dynamisch Aufrufe auf die URLs, unter denen die zugehörigen Bildkacheln K aus dem Speicher-Service-Modul 13 heruntergeladen werden können.

Die Anzeige der Bilddaten wird im Rahmen der Internet-Applikation 17 gesteuert von dem Layout-Manager 22, der hierzu das Szenenrendermodul 20 und das Methodenmodul 21 ansteuert. Das Szenenrendermodul 20 definiert einen anzuzeigenden Bildbereich. Für das Laden der für die Anzeige dieses Bildbereichs nötigen Bildkacheln K greift das Szenenrendermodul 20 wiederum auf das Kachellademodul 19 zurück. Das Kachellademodul 19 lädt diese Bildkacheln K aus dem Bildkachelspeicher 26 des im Datencenter 2 lokalisierten Speicher-Service-Moduls 13, indem es die den Bildkacheln K zugeordneten URLs dynamisch aufruft.

Das Szenenrendermodul 20 erstellt aus den geladenen Bildkacheln K eine Szene, d.h. eine dem anzuzeigenden Bildbereich entsprechende Gesamtdarstellung und zeigt diese Szene mittels einer Benutzerschnittstelle (User Interface, kurz: UI) am Client 3a bzw. 3d an.

Durch das Methodenmodul 21 werden dabei begleitend die eingangs genannten Methoden (Tools) zur Auswertung der angezeigten Bilddaten zur Verfügung gestellt und - bei Anforderung durch den Nutzer N - ausgeführt. Wie vorstehend erwähnt, umfassen diese Methoden insbesondere Methoden zur Navigation in den angezeigten Bilddaten, nämlich zum Verschieben des angezeigten Bildbereichs relativ zu der gesamten Bildfläche und zum Ändern der Bildauflösung (und somit zum Vergrößern oder Verkleinern des Bildbereichs).

Sofern der Nutzer N unter Nutzung dieser Methoden den angezeigten Bildbereich verschiebt, verkleinert oder vergrößert, werden durch das Kachellademodul 19 im nötigen Maße Bildkacheln K nachgeladen und von dem Szenenrendermodul 20 in die angezeigte Szene ergänzt.

Bildkacheln K, die im Zuge eines Anzeigevorgangs bereits in den Client 3a-3e geladen wurden, werden von dem Kachellademodul 19 in einem Cachespeicher des Clients 3a-3e zwischengespeichert. Bei erneutem Bedarf werden diese Bildkacheln K von dem Kachellademodul 19 nicht erneut aus dem Datencenter 2 geladen, sondern ressourcen- und zeitsparend aus dem Cache-speicher entnommen.

Befunde B, die der Nutzer N während der Ansicht der Bilddaten in den jeweiligen Client 3a bzw. 3d eingibt, werden von dem Befundmanager 24 gesammelt und - in regelmäßigen Zeitabschnitten, bevorzugt aber stets bei Beendigung der Internet-Applikation 17 - zur Sicherung an den Befundspeicher 29 des Speicher-Service-Moduls 13 übermittelt.

Sofern vorgesehen, wird durch den Double-Reading-Manager 25 ein Double-Reading-Schritt gestartet, sobald der Nutzer N einen Fall abgeschlossen hat und infolgedessen den Anzeigemodus der Internet-Applikation 17 schließt.

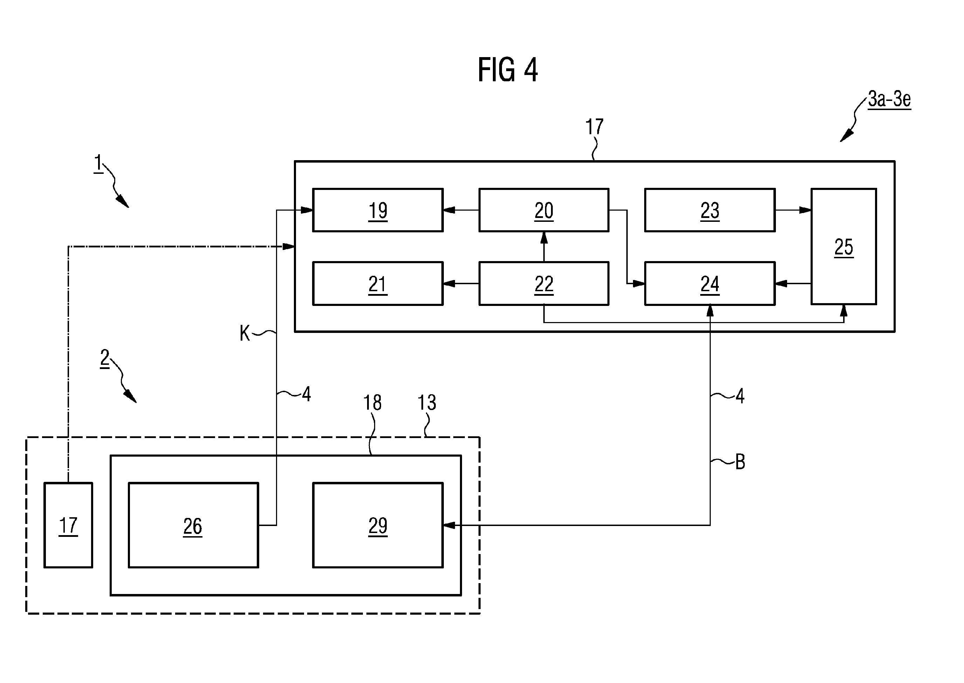

Wie aus der vorstehenden Beschreibung entnehmbar, und in FIG 4 graphisch verdeutlicht ist, ist die Business Logik der Einrichtung 1, die zum Anzeigen der Bilddaten und zur Verwaltung der Befunde B erforderlich ist, ausschließlich in der Internet-Applikation 17 implementiert. Die Internet-Applikation wird somit als RIA nach dem Download aus dem Datencenter 2 (wie in FIG 4 durch einen strichpunktierten Pfeil angedeutet ist) vollständig clientseitig ausgeführt, ohne dass sie für das (Nach-)laden von Bildkacheln K auf einen in dem Datencenter lokalisierten Web-Server zurückgreifen müsste. Es sind also für diese Zwecke in dem Datencenter 2 keine Dienste implementiert. Vielmehr erfolgt das Laden der Bildkacheln K ausschließlich durch Dateisystemaufrufe, die von dem Client 3a-3e kontrolliert werden.

Die Abwesenheit von im Data-Center 2 implementierten Rechendiensten für die Anzeige und Auswertung der Bilddaten ermöglicht eine hohe Perfomance sowie quasi unendliche Skalierbarkeit des Verfahrens und der zugehörigen Einrichtung 1.

Um die Kommunikation des Datencenters 2 mit den Clients 3a-3e so wenig wie möglich zu stören, arbeitet das Backend-Service-Modul 15 zeitlich getrennt von den übrigen Prozessen des Datencenters 2, insbesondere überwiegend während der Nachtzeiten. Zudem wird das Backend-Service-Modul 15 ausschließlich asynchron im Hintergrund ausgeführt, um Anfragen der Clients 3a-3e an das Datencenter 2 nicht zu verzögern. Das Frontend-Service-Modul 14 arbeitet dagegen synchron.

Bei der Einrichtung 1 handelt es sich um ein für die Verwendung in der Pathologie oder alternativ für die Verwendung in der Mammographie spezialisiertes System. Entsprechend handelt es sich bei dem Eingangsbilddatensatz E um medizinische Bilddaten, wie sie typischerweise in der Pathologie bzw. Mammographie zu Befundungszwecken herangezogen werden.

### Bezugszeichenliste

- 1: Einrichtung
- 2: Datencenter
- 3a-e: Client
- 4: Datenkommunikationsnetz
- 5: LAN
- 6: LAN
- 7: W-LAN
- 8: Router
- 9: Internet
- 10: Firewall
- 11: LAN
- 12a-c: Modalität
- 13: Speicher-Service-Modul
- 14: Frontend-Service-Modul
- 15: Backend-Service-Modul
- 16: Datenbank-Service-Modul
- 17: Internet-Applikation
- 18: Kontextspeicher
- 19: Kachellademodul
- 20: Szenenrendermodul
- 21: Methodenmodul
- 22: Layout-Manager
- 23: Worklist-Manager
- 24: Befund-Manager
- 25: Double-Reading-Manager
- 26: Bildkachelspeicher
- 27: DICOM-Bildspeicher
- 28: Vorverarbeitungsmodul
- 29: Befundspeicher
- 30: DICOM-Empfangsmodul
- 31: DICOM-Sendemodul
- 32: (DICOM/JPEG-)Konverter
- 33: Datenbank-Worklistmanager
- 34: Datenbank-Bildmanager
- 35: Datenbank-Zugriffsmanager
- 36: Port-Mediator
- B: Befund
- E: (Eingangs-)Bilddatensatz
- I: Indexinformation
- K: Bildkachel
- N: Nutzer
- S: Sekundärbild

## Patentansprüche

1. Verfahren zur Anzeige von medizinischen Bilddaten,
- bei welchem in einem zentralen Datencenter (2) aus mindestens einem medizinischen Eingangsbilddatensatz (E), der in einem dem DICOM-Standard entsprechenden Format oder einem vergleichbaren Datenformat für medizinische Bilddaten vorliegt, eine Hierarchiegruppe von pyramidentransformierten Bildkacheln (K) erzeugt wird, wobei die Bildkacheln (K) in einem Bildkachelspeicher (26) des Datencenters (2) hinterlegt werden,
- bei welchem jeder Bildkachel (K) eine URL zugewiesen wird, die eine Information über die Bildauflösung der Bildkachel (K) und den von Ihr abgedeckten Bildausschnitt enthält,
- bei welchem an einem mit dem Datencenter (2) datenkommunikationstechnisch verbundenen Client (3a-3e) mittels eines Web-Browsers eine Internet-Applikation (17) ausgeführt wird, wobei durch die Internet-Applikation (17) ein anzuzeigender Bildbereich sowie eine zugehörige Bildauflösung bestimmt werden, und
- bei welchem durch die Internet-Applikation (17) die URL der oder jeder Bildkachel (K) mit entsprechender Bildauflösung ermittelt wird, deren Bildausschnitt mit dem anzuzeigenden Bildbereich überlappt,
- und bei welchem durch die Internet-Applikation (17) unter Zugriff auf die oder jede bestimmte URL die zugehörigen Bildkacheln (K) aus dem Bildkachelspeicher (26) in den Client (3a-3e) geladen und dort angezeigt werden.

2. Verfahren nach Anspruch 1,
bei welchem die Bildkacheln (K) in dem Datencenter (2) in einem komprimierten Bilddatenformat, insbesondere JPEG oder PNG, erzeugt werden.

3. Verfahren nach Anspruch 1 oder 2,
bei welchem zu dem oder jedem Eingangsbilddatensatz (E) in dem Datencenter (2) Indexinformation (I) erzeugt und hinterlegt wird, die den Speicherort der zugehörigen Bildkacheln (K), sowie mit dem Eingangsbilddatensatz (E) verknüpfte Angaben enthält, insbesondere Angaben zu der Modalität, dem Patienten, dem Befundungsstatus und/oder dem zugeordneten Arzt.

4. Verfahren nach einem der Ansprüche 1 bis 3,
bei welchem bereits in den Client (3a-3e) geladene Bildkacheln (K) während der Ausführung der Internet-Applikation (17) in einem Cachespeicher des Clients (3a-3e) für einen erneuten Zugriff zwischengespeichert werden.

5. Verfahren nach einem der Ansprüche 1 bis 4,
bei welchem das Laden der Bildkacheln (K) aus dem Bildkachelspeicher (26) durch die Internet-Applikation (17) mittels asynchroner Kommunikation vorgenommen wird.

6. Verfahren nach einem der Ansprüche 1 bis 5, wobei die Internet-Applikation (17) zu ihrer Ausführung von dem Web-Browser aus dem Datencenter (2) heruntergeladen wird.

7. Verfahren nach einem der Ansprüche 1 bis 6,
bei welchem durch die Internet-Applikation (17) zur zusätzlichen Unterstützung der Bildauswertung über eine Nutzerschnittstelle mindestens eine Bildauswertungs-Methode zur Verfügung gestellt wird, insbesondere mindestens eine Methode zur Vermessung der Bildinformation, zur Markierung und/oder Beschreibung von Befunden (B) und/oder zu Beschriftung der Bildinformation.

8. Verfahren nach einem der Ansprüche 1 bis 7,
wobei durch die Internet-Applikation (17) zur zusätzlichen Unterstützung der Bildauswertung Befunde (B), die von einem Client-Nutzer (N) markiert und/oder eingegeben worden sind, gesammelt und in einem Befundspeicher (29) des Datencenters (2) hinterlegt werden.

9. Verfahren nach einem der Ansprüche 1 bis 8,
wobei durch die Internet-Applikation (17) über eine Nutzerschnittstelle Navigationsmittel zur Verfügung gestellt werden, mittels derer ein Client-Nutzer (N) den anzuzeigenden Bildbereich reversibel verschieben und vergrößern oder verkleinern kann.

10. Einrichtung (1) zur Anzeige von medizinischen Bilddaten, mit einem zentralen Datencenter (2), der ein Backend-Service-Modul (15) und ein einen Bildkachelspeicher (26) enthaltendes Speicher-Service-Modul (13) umfasst, sowie mit einem Client (3a-3e), der über ein Datenkommunikationsnetz (4) mit dem Datencenter (2) verbunden ist, und in dem ein Web-Browser implementiert ist,
- wobei das Backend-Service-Modul (15) dazu eingerichtet ist, aus mindestens einem medizinischen Eingangsbilddatensatz (E), der in einem dem DICOM-Standard entsprechenden Format oder einem vergleichbaren Datenformat vorliegt, eine Hierarchiegruppe von pyramidentransformierten Bildkacheln (K) zu erzeugen, und die Bildkacheln (K) in dem Bildkachelspeicher (26) zu hinterlegen,
- wobei das Backend-Service-Modul (15) dazu eingerichtet ist, jeder Bildkachel (K) eine URL zuzuweisen, die eine Information über die Bildauflösung der Bildkachel (K) und den von Ihr abgedeckten Bildausschnitt enthält,
- wobei in dem Speicher-Service-Modul (13) eine Internet-Applikation (17) zum Herunterladen auf den Client (3a-3e) hinterlegt ist,
- wobei die Internet-Applikation (17) mittels des clientseitig implementierten Web-Browsers aus dem Speicher-Service-Modul (13) herunterladbar und ausführbar ist,
- wobei die Internet-Applikation (17) dazu eingerichtet ist, während der Ausführung einen anzuzeigenden Bildbereich sowie eine zugehörige Bildauflösung zu bestimmen, die URL der oder jeder Bildkachel (K) mit entsprechender Bildauflösung zu ermitteln, deren Bildausschnitt mit dem anzuzeigenden Bildbereich überlappt, und unter Zugriff auf die oder jede bestimmte URL die zugehörigen Bildkacheln (K) aus dem Bildkachelspeicher (16) in den Client (3a-3e) zu laden und dort anzuzeigen.

11. Einrichtung (1) nach Anspruch 10,
wobei das Backend-Service-Modul (15) dazu eingerichtet ist, die Bildkacheln (K) in einem komprimierten Bilddatenformat, insbesondere JPEG oder PNG, zu erzeugen.

12. Einrichtung (1) nach Anspruch 10 oder 11,
wobei das Backup-Service-Modul (15) dazu eingerichtet ist, zu dem oder jedem Eingangsbilddatensatz (E) Indexinformation (I) zu erzeugen und zu hinterlegen, die den Speicherort der zugehörigen Bildkacheln (K), sowie mit dem Eingangsbilddatensatz (E) verknüpfte Angaben enthält, insbesondere Angaben zu der Modalität, dem Patienten, dem Befundungsstatus und/oder dem zugeordneten Arzt.

13. Einrichtung (1) nach einem der Ansprüche 10 bis 12, wobei die Internet-Applikation (17) dazu eingerichtet, bereits in den Client (3a-3e) geladene Bildkacheln (K) in einem Cachespeicher des Clients (3a-3e)für einen erneuten Zugriff zwischenzuspeichern.

14. Einrichtung (1) nach einem der Ansprüche 10 bis 13, wobei die Internet-Applikation (17) dazu eingerichtet ist, das Laden der Bildkacheln (K) aus dem Bildkachelspeicher (26) mittels asynchroner Kommunikation vorzunehmen.

15. Einrichtung (1) nach einem der Ansprüche 10 bis 14, wobei die Internet-Applikation (17) in dem Speicher-Service-Modul (13) hinterlegt ist, so dass sie zu ihrer Ausführung von dem Web-Browser aus dem Speicher-Service-Modul (13) herunterladbar ist.

16. Einrichtung (1) nach einem der Ansprüche 10 bis 15, wobei die Internet-Applikation (17) dazu eingerichtet ist, zur zusätzlichen Unterstützung der Bildauswertung über eine Nutzerschnittstelle mindestens eine Bildauswertungs-Methode zur Verfügung zu stellen, insbesondere mindestens eine Methode zur Vermessung der Bildinformation, mindestens eine Methode zur Markierung und/oder Beschreibung von Befunden und/oder Beschriftung der Bildinformation.

17. Einrichtung (1) nach einem der Ansprüche 10 bis 16, wobei die Internet-Applikation (17) dazu eingerichtet ist, zur zusätzlichen Unterstützung der Bildauswertung Befunde (B), die von einem Client-Nutzer (N) markiert und/oder eingegeben worden sind, zu sammeln und in einem Befundspeicher (29) des Datencenters (2) zu hinterlegen.

18. Einrichtung (1) nach einem der Ansprüche 10 bis 16, wobei die Internet-Applikation (17) dazu eingerichtet ist, über eine Nutzerschnittstelle Navigationsmittel zur Verfügung zu stellen, mittels derer ein Client-Nutzer (N) den anzuzeigenden Bildbereich reversibel verschieben und vergrößern oder verkleinern kann.

19. Verwendung des Verfahrens nach einem der Ansprüche 1 bis 9 oder der Einrichtung (1) nach einem der Ansprüche 10 bis 18 zur Anzeige von Pathologie-Bilddaten.

20. Verwendung des Verfahrens nach einem der Ansprüche 1 bis 9 oder der Einrichtung (1) nach einem der Ansprüche 10 bis 18 zur Anzeige von Mammographie-Bilddaten.

## Geänderte Patentansprüche

### Geänderte Patentansprüche gemäss Regel 137(2) EPÜ.

**1.** Verfahren zur Anzeige von medizinischen Bilddaten,
- bei welchem in einem zentralen Datencenter (2) aus mindestens einem medizinischen Eingangsbilddatensatz (E), der in einem dem DICOM-Standard entsprechenden Format oder einem vergleichbaren Datenformat für medizinische Bilddaten vorliegt, eine Hierarchiegruppe von pyramidentransformierten Bildkacheln (K) erzeugt wird, wobei die Bildkacheln (K) in einem Bildkachelspeicher (26) des Datencenters (2) hinterlegt werden,
- bei welchem jeder Bildkachel (K) eine URL zugewiesen wird, die eine Information über die Bildauflösung der Bildkachel (K) und den von Ihr abgedeckten Bildausschnitt enthält,
- bei welchem an einem mit dem Datencenter (2) datenkommunikationstechnisch verbundenen Client (3a-3e) mittels eines Web-Browsers eine Internet-Applikation (17) ausgeführt wird, wobei ausschließlich durch die Internet-Applikation (17) ein anzuzeigender Bildbereich sowie eine zugehörige Bildauflösung bestimmt werden, und
- bei welchem ausschließlich durch die Internet-Applikation (17) die URL der oder jeder Bildkachel (K) mit entsprechender Bildauflösung ermittelt wird, deren Bildausschnitt mit dem anzuzeigenden Bildbereich überlappt,
- und bei welchem durch die Internet-Applikation (17) unter Zugriff auf die oder jede bestimmte URL die zugehörigen Bildkacheln (K) aus dem Bildkachelspeicher (26) in den Client (3a-3e) geladen und dort angezeigt werden.

**10.** Einrichtung (1) zur Anzeige von medizinischen Bilddaten, mit einem zentralen Datencenter (2), der ein Backend-Service-Modul (15) und ein einen Bildkachelspeicher (26) enthaltendes Speicher-Service-Modul (13) umfasst, sowie mit einem Client (3a-3e), der über ein Datenkommunikationsnetz (4) mit dem Datencenter (2) verbunden ist, und in dem ein Web-Browser implementiert ist,
- wobei das Backend-Service-Modul (15) dazu eingerichtet ist, aus mindestens einem medizinischen Eingangsbilddatensatz (E), der in einem dem DICOM-Standard entsprechenden Format oder einem vergleichbaren Datenformat vorliegt, eine Hierarchiegruppe von pyramidentransformierten Bildkacheln (K) zu erzeugen, und die Bildkacheln (K) in dem Bildkachelspeicher (26) zu hinterlegen,
- wobei das Backend-Service-Modul (15) dazu eingerichtet ist, jeder Bildkachel (K) eine URL zuzuweisen, die eine Information über die Bildauflösung der Bildkachel (K) und den von Ihr abgedeckten Bildausschnitt enthält,
- wobei in dem Speicher-Service-Modul (13) eine Internet-Applikation (17) zum Herunterladen auf den Client (3a-3e) hinterlegt ist,
- wobei die Internet-Applikation (17) mittels des clientseitig implementierten Web-Browsers aus dem Speicher-Service-Modul (13) herunterladbar und ausführbar ist,
- wobei die Internet-Applikation (17) dazu eingerichtet ist, während der Ausführung einen anzuzeigenden Bildbereich sowie eine zugehörige Bildauflösung zu bestimmen, die URL der oder jeder Bildkachel (K) mit entsprechender Bildauflösung zu ermitteln, deren Bildausschnitt mit dem anzuzeigenden Bildbereich überlappt, und unter Zugriff auf die oder jede bestimmte URL die zugehörigen Bildkacheln (K) aus dem Bildkachelspeicher (16) in den Client (3a-3e) zu laden und dort anzuzeigen, und
- wobei die Internet-Applikation (17) die gesamte Funktionalität enthält, die zum Einlesen der Bildkacheln (K) inklusive des dynamischen Nachladens benötigt wird.
